# EUROPEAN PATENT APPLICATION

(11) **EP 4 555 936 A1**
(43) Date of publication of application: **21.05.2025**
(21) Application number: 23210804.3
(22) Date of filing: 20.11.2023
(51) Int. Cl.: A61B 6/03, A61B 6/00, A61B 6/58, G01T 1/00

(54) **X-RAY IMAGING WITH IN-LINE CALIBRATION**

(71) Applicant: Koninklijke Philips N.V., 5656 AG Eindhoven (NL)
(72) Inventor: ELENBAAS, Thijs, Eindhoven (NL); VAN DER WACHT, Rogier, Eindhoven (NL); DRIES, Johan Juliana, 5656 AG Eindhoven (NL); GAST, Tomas, Eindhoven (NL)
(74) Representative: Philips Intellectual Property & Standards

(57) **Abstract**

The present invention relates to calibration for X-ray imaging. In order to provide facilitated calibration of an X-ray imager, an X-ray source (10) for X-ray imaging with in-line calibration is provided. The X-ray source comprises a source housing (12), an X-ray generation unit (14) and a calibration pattern (16). The X-ray generation unit is arranged within the source housing and is configured to generate an X-ray beam (18) of multi-spectral radiation for spectral imaging along a beam path (20). The housing comprises an X-ray aperture (22) for transmitting the X-ray beam along the X-ray beam path towards an X-ray detector. The calibration pattern is arranged at the source housing within the X-ray beam path. The calibration pattern is a 2D geometrical pattern (24) made from an X-ray absorbing material. Further, the 2D geometrical pattern comprises a plurality of linear elements (26) arranged in predetermined configuration allowing an image-based deduction of at least one of the group of: distance, inclination and distortion between a detector plane and the X-ray source.

## Description

### FIELD OF THE INVENTION

The present invention relates to calibration of an X-ray imager, e.g. to X-ray imaging with in-line calibration; the present invention relates in particular to an X-ray source for X-ray imaging with in-line calibration, to an X-ray imaging system, to a calibration kit for X-ray imaging systems and to a method for calibration during X-ray imaging.

### BACKGROUND OF THE INVENTION

In X-ray imaging, a precise geometrical arrangement is required for a number of imaging procedures for certain tasks. For example, for creating DSA's, 2D roadmaps or for CBCT 3D reconstructions, a precise source-detector calibration may be required. However, it has been shown that regular calibration may be cumbersome to perform, e.g. in view of staff workload.

### SUMMARY OF THE INVENTION

There may thus be a need for facilitated calibration for X-ray imaging.

The object of the present invention is solved by the subject-matter of the independent claims; further embodiments are incorporated in the dependent claims. It should be noted that the following described aspects of the invention apply also for the X-ray source for X-ray imaging with in-line calibration, for the X-ray imaging system, for the calibration kit for X-ray imaging systems and for the method for calibration during X-ray imaging.

According to the present invention, an X-ray source for X-ray imaging with in-line calibration is provided. The X-ray source comprises a source housing, an X-ray generation unit and a calibration pattern. The X-ray generation unit is arranged within the source housing and is configured to generate an X-ray beam of multi-spectral radiation for spectral imaging along a beam path. The housing comprises an X-ray aperture for transmitting the X-ray beam along the X-ray beam path towards an X-ray detector. The calibration pattern is arranged at the source housing within the X-ray beam path. The calibration pattern is a geometrical pattern made from an X-ray absorbing material. The geometrical pattern comprises a plurality of graphical elements arranged in predetermined configuration.

As an effect, calibration is provided without additional X-ray dose, but during an actual imaging procedure. Due to providing spectral imaging, the respective information suitable for calibration can be derived from the X-ray image data retrieved.

As an advantage, an option for a live calibration is provided that addresses potential issues that may arise in X-ray imaging systems which do not have a C-arm for holding source and detector, but which have lighter and less bulky holding arrangements like robot arms. As an example, if the ceiling and the floor are such that robotic arms mounting X-ray source and detector to the floor and ceiling respectively are not optimally fixated with respect to each other, the live calibration allows to detect a possible misalignment and to at least partly take this into account for further image processing steps.

X-ray imaging systems which do not have a C-arm are also referred to as C-less setups. One aspect in which the C-less setup is different from a traditional C-arm is that the source and detector are not directly fixated to each other. While a C-arm can exhibit some deformation, this is very limited, and it is reproducible which allows consideration in imaging processing steps.

However, in a C-less setup, as the source and detector are connected to the floor and ceiling respectively, spatial coherency is partly dependent on the room as a factor outside of the system. This means that there may be a significant variation in coherency between one installation and the other, and spatial errors may not always be reproducible. This is where the live calibration provides advantageous improvement in facilitating the workflow as well as improving precision.

As an effect, it is of advantage to do the calibration on the actual patient data. Even though this introduces some sort of image artifacts, it is possible to remove these due to spectral imaging. As an example, a live calibration results in improved calibration over e.g. regular calibration prior to the procedure. Live calibration is suitable for cases where pre-calibration based on some calibration scheme over time would not result in sufficient accuracy, for example due non-reproducible errors between calibration and patient scan.

In an example, the material of the geometrical pattern is spectrally distinctive from human anatomy, allowing an image-based deduction of at least one of the group of distance, inclination and distortion between a detector plane and the X-ray source. Accordingly, the calibration pattern may remain in place during operation of the X-ray imaging system, whereby spectral imaging processing such as a material decomposition algorithm may separate the calibration pattern in the X-ray images from the imagery of the anatomy under examination.

According to an example, the calibration pattern is fixedly arranged at the source housing within the X-ray path.

According to an example, the geometrical pattern comprises a 2D geometric pattern such as a plurality of linear elements arranged in predetermined configuration. For example, the 2D geometrical pattern is a square-based line pattern with two crossing axes of symmetry. A plurality of linear segments forms a plurality of open field segments.

Alternatively, a 2D geometric pattern is provided comprising a plurality of dots or pixels arranged in a predetermined configuration. As yet another alternative, instead of a 2D geometric pattern, a 3D structure may be envisaged as the geometric pattern.

In an option, the geometrical pattern is made from tungsten elements, for example in the form of tungsten filaments. The tungsten elements are distinctive from human anatomy in spectral X-ray imaging. In particular, the tungsten material of the geometrical pattern may be distinguished from tissue, bones and water for example by means of processing the spectral image data by a material decomposition algorithm.

According to a further aspect, also an X-ray imaging system is provided.

The X-ray imaging system comprises an X-ray source according to one of the preceding examples, an X-ray detector, a holding arrangement for the X-ray source and the X-ray detector and an image data processing unit. The X-ray source and the X-ray detector are mounted to the holding arrangement. The X-ray source is configured to provide spectral imaging X-ray radiation for imaging an object of interest arranged between the X-ray source and the X-ray detector. The X-ray detector is configured to receive X-ray radiation after at least partly passing through the object of interest and to detect spectral imaging data, the spectral imaging data comprising image portions representing the calibration pattern. The image data processing unit is configured to identify, based on the detected spectral imaging data, the image portions representing the calibration pattern and to segment the calibration pattern within the image data based on the spectral imaging data. The image data processing unit is configured to provide the segmented calibration pattern for further geometrical calibration computation steps.

According to an example, the holding arrangement comprises a robot arrangement with a source robot arm and a detector robot arm. The X-ray source is mounted to the source robot arm, and the X-ray detector is mounted to the detector robot arm.

According to an example, the imaging system is configured for calibration during X-ray imaging on a regular pattern. In an option, calibration is provided during every X-ray imaging run.

According to an example, the X-ray source is configured to provide X-ray radiation of at least a first spectrum and a second spectrum. The X-ray detector is configured to detect first image data relating to the first spectrum and second image data relating to the second spectrum. The image data processing unit is configured to combine the first image data and the second image data for a detection of the calibration pattern in the image data.

According to an example, in a first option, the X-ray source is configured to provide multispectral imaging and the X-ray detector is a multilayer detector configured to detect different X-ray energy levels. In an alternative second option, the X-ray source is configured to provide radiation based on switching voltages and the X-ray detector is configured to detect different X-ray energy levels in a temporally separated manner.

According to the present invention, also a calibration kit for X-ray imaging systems is provided. The kit comprises a calibration pattern and an image data processing unit. The calibration pattern is a geometrical pattern made from an X-ray absorbing material. The geometrical pattern comprises a plurality of graphical elements configured to be arranged in predetermined configuration allowing an image-based deduction of at least one of the group of: distance, inclination and distortion between a detector plane and the X-ray source. The calibration pattern is configured to be arranged at a source housing within an X-ray path of an X-ray generation unit that is arranged within the source housing and that is configured to generate an X-ray beam of multi-spectral radiation for spectral imaging. As an option, the calibration pattern is configured to be fixedly arranged at the source housing. The housing comprises an X-ray aperture for transmitting the X-ray beam along an X-ray path towards an X-ray detector. The image data processing unit is configured to identify image portions detected by the detector, which image portions represent the calibration pattern, and to segment the calibration pattern within the image data based on the spectral imaging data. The image data processing unit is configured to provide the segmented calibration pattern for further geometrical calibration computation steps.

According to an option of the kit, the 2D geometrical pattern comprises a plurality of linear elements arranged in predetermined configuration.
According to the present invention, also a method for calibration during X-ray imaging is provided. The method comprises the following steps:
- arranging a calibration pattern within an X-ray beam path; the calibration pattern is a 2D geometrical pattern made from an X-ray absorbing material;
- generating X-ray radiation of multi-spectral radiation for spectral imaging along the beam path by an X-ray source towards a detector; an object is arranged between the X-ray source and the X-ray detector;
- detecting spectral X-ray radiation by the detector providing detected image data;
- determining the geometrical pattern in the detected image data;
- based on the determined geometrical pattern, deducting of at least one data of the group of distance, inclination and distortion between a detector plane and the X-ray source; and
- providing the deducted data for calibration purposes.
According to an option of the method, the geometrical pattern comprises a plurality of linear elements arranged in predetermined configuration.

According to an aspect, the X-ray system itself is used to perform calibration measurements when the subject, e.g. a patient, is on the subject support, e.g. the patient table. By combining the calibration pattern on the patient image, regulations are addressed that require that all radiation must be used for imaging. While it may be difficult to remove a calibration pattern from a subject in a standard X-ray image, the provision of spectral imaging improves detectability and thus achieves sufficient improvement for removing the pattern at least partly.

To use multi-spectral imaging allows to distinguish the calibration pattern component from the patient component in X-ray images, for example by applying suitable material decomposition algorithms to the spectral X-ray image data separating the calibration pattern material from materials commonly found in a patient's anatomy, such as water, bones and (soft) tissue. In this manner, more information is available than just changes in Hounsfield values following a predefined geometric pattern. This allows for a) more effective (subpixel) segmentation of the calibration pattern and subsequent high-precision geometric compensation, and for b) more effectively removing the calibration pattern from the (multi-spectral) X-ray image of the patient with fewer artifacts.

A field of application is basically any X-ray system that requires on-the-fly calibration and that can perform multi-spectral imaging. Examples are interventional imaging systems; however, the present disclosure is not limited thereto and the live calibration described herein may also be applicable in diagnostic imaging systems, such as CT or SPECT/PET-CT imaging systems.

According to an aspect, the relative position between X-ray tube (source) and detector can accurately be measured based on the spectral imaging data and it is hence possible to reduce a calibration effort by service also for 3D reconstruction calibrations and roadmapping calibrations.

In an option, multi-spectral imaging is used to distinguish the calibration pattern component from the patient component in spectral X-ray images, and thereby more precisely track the calibration pattern and perform better compensation. Based on the spectral image information, the calibration pattern can be removed from the image of the patient with fewer artifacts.

In an option, multi-spectral calibration is performed using a switching kV-source. For example, fluoro imaging or X-ray exposure at low kV is performed. Further, fluoro imaging or X-ray exposure at high kV is performed. Then, material values are calculated and separated based on absorption profile at different kV values. The material pattern is then used for geometrical correction. Further, the calibration pattern is subtracted from the X-ray patient image.

In another option, a multi-spectral, e.g. dual layer, X-ray detector is provided with an X-ray source with a sufficiently broad spectrum. In this case, no X-ray switching is necessary, but calculating the different absorption profiles of the calibration pattern works in the same manner.

In an example, from the multi-spectral image data, the material of the geometric pattern is distinguished from an object of interest, such as a human anatomy, by means of processing the image data with a suitable material decomposition algorithm.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of an X-ray source for X-ray imaging with in-line calibration.
Fig. 2 shows an example of a calibration pattern provided in the X-ray source of Fig. 1.
Fig. 3 shows an illustration of another example of an X-ray source with the calibration pattern of Fig. 2.
Fig. 4a and Fig. 4b show different image data of spectral imaging in which the calibration pattern is indicated in the context of an anatomical region of interest of a subject.
Fig. 5 schematically shows an example of an X-ray imaging system with optional robot arms for movably holding source and detector.
Fig. 6 shows basic steps of an example of a method for calibration during X-ray imaging.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

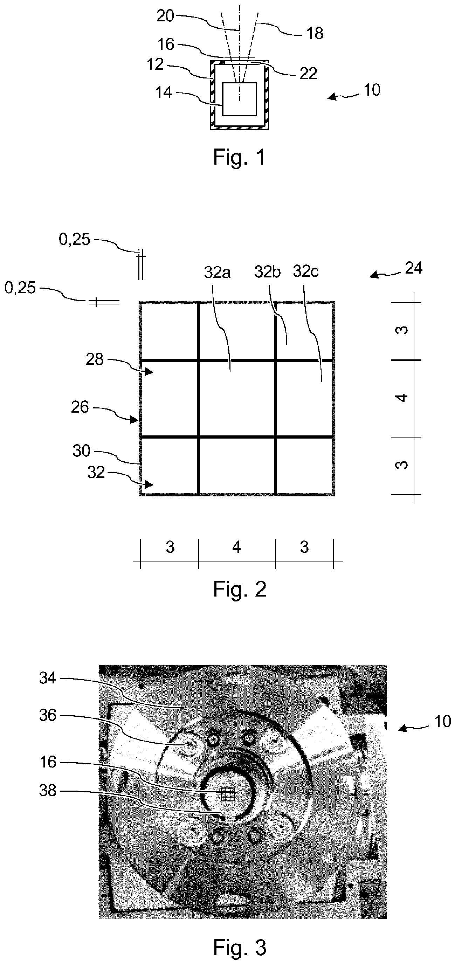

Fig. 1 schematically shows an example of an X-ray source 10 for X-ray imaging with in-line calibration. The X-ray source 10 comprises a source housing 12, an X-ray generation unit 14 and a calibration pattern 16. The X-ray generation unit 14 is arranged within the source housing 12 and is configured to generate an X-ray beam 18 of multi-spectral radiation for spectral imaging along a beam path 20. The source housing 12 comprises an X-ray aperture 22 for transmitting the X-ray beam 18 along the X-ray beam path 20 towards an X-ray detector (not shown in Fig. 1). The calibration pattern 16 is arranged at the source housing 12 within the X-ray beam path 20. The calibration pattern 16 is a geometrical pattern 24 (see e.g. Fig. 2) made from an X-ray absorbing material. The geometrical pattern 24 in this example is a 2D geometrical pattern that comprises a plurality of graphical elements arranged in predetermined configuration. As shown, the exemplary 2D geometrical pattern comprises a plurality of linear elements 26 arranged in predetermined configuration.

The term "source housing" relates to an enclosure encapsulating the X-ray generation device, also referred to as the X-ray source. The source housing 12 may be provided as radio-opaque enclosure or envelope.

The term "X-ray generation unit" relates to the source or tube providing the X-ray radiation.

The term "calibration pattern" relates to an arrangement provided for achieving exact calibration information.

The term "aperture" relates to a part of the source housing 12 that is transparent, or only dampening with a minimum effect, for the spectral radiation generated by the X-ray source, i.e. the X-ray generation unit 14.

The term "geometrical pattern" relates to a graphical or otherwise generated pattern that is characterized by simple and unambiguously identifiable forms different to what can be expected when radiating the object.

The term "linear elements" relates to parts or portions that are provided as line-segments.

In an example, the calibration pattern 16 is permanently fixed within the X-ray path 20. The provided calibration is suitable for X-ray systems in which deviations from the planned geometric path are not reproducible and as such cannot be removed by pre-calibration. The e.g. live calibration together with the patient in view allows to at least mitigate the off-calibration effect.

The use of the calibration pattern 16 can be seen by a detector in the X-ray field, allowing distinguishing, in a multispectral field, the calibration pattern 16 with absorption characteristics different from, blood, bone, soft-tissue, and iodine. This enables image-based deduction of at least one of the group of distance, inclination and distortion between a detector plane and the X-ray source 10.

In an option of the example of Fig. 1, the calibration pattern 16 is fixedly arranged at the source housing 12 within the X-ray path 20.

In an alternative option, the calibration pattern 16 is removably arranged at the source housing 12 within the X-ray path 20.

Fig. 2 shows an example of the calibration pattern 16 provided in the X-ray source 10 of Fig. 1.

In an option, the geometrical pattern 16 is a square-based line pattern 28 with two crossing axes of symmetry. A plurality of linear segments 30 forms a plurality of open field segments 32.

The term "square-based line pattern" relates to a grid-like arrangement based on rectangular forms, like based on a square basic form.

In an example, a central open field segment 32a is provided surrounded by peripheral field segments, corner segments 32b and middle segments 32c. For example, the peripheral field segments are having at least two different sizes.

In an example, the central open field segment 32a is square-shaped; the corner segments 32b of the peripheral field segments are also square-shaped, and the middle segments 32c of the peripheral field segments are rectangular-shaped.

As an example, the central open field 32a and the corner segments 32b have an edge-length ratio of 4 : 3.

As an example, a grid dimension of 10 x 10 mm is provided. The central open field 32a is dimensioned as 4 mm x 4 mm while the corner segments 32b are dimensioned as 3 mm x 3 mm. The middle segments 32c of the peripheral field segments are dimensioned as 3 mm x 4 mm.

In an example, the calibration pattern 16 is made from filament having a diameter of 0.25 mm and the pattern is provided with an outer grid dimension of approximately 10 mm. With a 0.25 mm filament, the outer dimensions are 10.25 mm.

In another option, the 2D geometrical pattern 16 is a triangular-based line pattern with three crossing axes of symmetry (not shown).

In a further option, the 2D geometrical pattern 16 is a circular-based line pattern with at least two crossing axes of symmetry (also not shown).

In case of an offset in distance, the size of the calibration pattern 16 is a calibration indicator.

In case of an offset in inclination, the deviation of the form of pattern is a calibration indicator.

The calibration pattern 16 allows to determine an off for a deviation of at least one of the group of X-, Y- and Z-position, and at least one of the group of X-, Y- and Z-rotation with respect to the X-ray source as well as the X-detector.

In an example, provided as an option, the 2D geometrical pattern 16 is made from materials spectrally distinctive from human anatomy.

In another option, the 2D geometrical pattern 16 is made from Tungsten filaments.

In an option, not shown in detail, an X-ray imaging device is provided that comprises an X-ray source according to one of the preceding examples, an X-ray detector and an image data processing unit. The X-ray source is configured to provide spectral imaging X-ray radiation for imaging an object of interest arranged between the X-ray source and the X-ray detector. The X-ray detector is configured to receive X-ray radiation after at least partly passing through the object of interest; and to detect spectral imaging data, the spectral imaging data comprising image portions representing the calibration pattern. The image data processing unit is configured to identify the image portions representing the calibration pattern and to segment the calibration pattern within the image data based on the spectral imaging data. The image data processing unit is configured to provide the segmented calibration pattern for further geometrical calibration computation steps.

Fig. 3 shows an illustration of another example of the X-ray 10 source with the calibration pattern 16 of Fig. 2. A tube-like X-ray absorbing housing 34 is shown with mounting and assembly screws 36. A center portion 38 is provided as an X-ray window for emitting X-ray radiation. An example of calibration pattern 16 is arranged in the center of the center portion 38 forming the X-ray window. Hence, calibration pattern 16 is arranged within a possible beam path when X-ray radiation is generated inside the housing 34. The X-ray 10 source provides spectral imaging for imagining a region of interest of a subject.

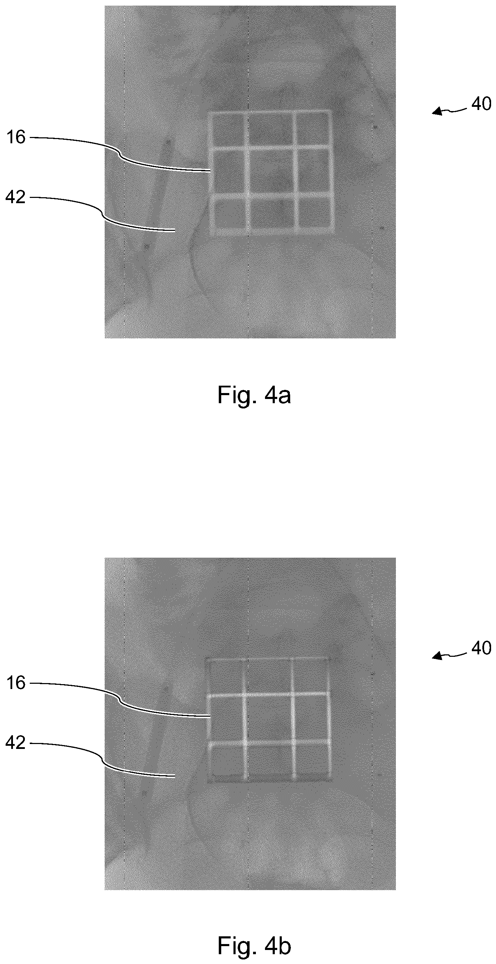

Fig. 4a and Fig. 4b show different image data 40 of spectral imaging in which the calibration pattern 16 is indicated in the context of an anatomical region of interest of a subject. The spectral image data shows an anatomical structure 42 such as tissue structures, organs, vessels or bone structures 42. As an example, Fig. 4a shows image data of the same region of interest as Fig. 4b; the image content of Fig. 4a is relating to a first spectral range of X-ray radiation and Fig. 4b shows image data relating to a second spectral range of X-ray radiation. As can be seen, both the anatomical structure 42 and the calibration pattern 16 are each visible in different ways in the two images. By combining the spectral image data, it is possible to identify the calibration pattern 16 in an enhanced manner and to then determine calibration data, e.g. in terms of distance, inclination and the like. In an option, it is also provided to segment and subtract the calibration pattern 16 from the rest of the image to provide improved image data of the anatomical structure 42.

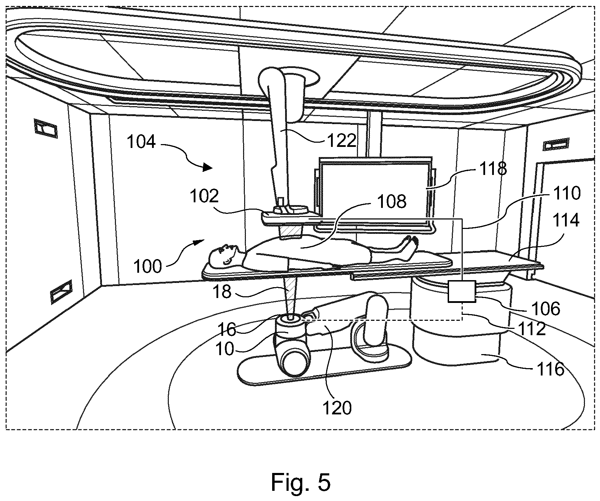

Fig. 5 schematically shows an example of an X-ray imaging system 100. The X-ray imaging system 100 comprises an example of the X-ray source 10 according to one of the examples above, an X-ray detector 102, a holding arrangement 104 for the X-ray source 10 and the X-ray detector 102 and an image data processing unit 106. The X-ray source 10 and the X-ray detector 102 are mounted to the holding arrangement 104. The X-ray source 10 is configured to provide spectral imaging X-ray radiation for imaging an object of interest arranged between the X-ray source 10 and the X-ray detector 102. The X-ray detector 102 is configured to receive X-ray radiation after at least partly passing through an object of interest 108 and to detect spectral imaging data, the spectral imaging data comprising image portions representing the calibration pattern. Image data processing unit 106 is configured to identify the image portions representing the calibration pattern 16 and to segment the calibration pattern within the image data based on the spectral imaging data. The image data processing unit 106 is also configured to provide the segmented calibration pattern for further geometrical calibration computation steps.

For example, image data processing unit 106 is connected to the X-ray detector 102 with a data connection 110. As an option, a further data connection 112 can be arranged to data-connect the X-ray source 10 with the image data processing unit 106.

The object of interest 108, e.g. a subject, may be arranged on a movable subject support 114 attached to a base 116, as shown in Fig. 5. Further, a display arrangement 118 is provided in the vicinity of the subject support 114.

The term "subject" may also be referred to as individual. The "subject" may further also be referred to as patient, although it is noted that this term does not indicate whether any illness or disease is actually present with the subject.

The term "holding arrangement" relates to providing a movable mount for the X-ray source 10 and the X-ray detector 102 of the X-ray imaging system 100.

Based on spectral imaging, relative absorption can be detected in the image, and this allows to distinguish contrast based on the calibration pattern from bones.

In an example, shown in Fig. 5 as an option, the holding arrangement 104 comprises a robot arrangement with a source robot arm 120 and a detector robot arm 122. X-ray source 10 is mounted to the source robot arm 120 and the X-ray detector 104 is mounted to the detector robot arm 122.

The term "robot arm" relates to a robot for moving and holding the X-ray source 10 or the X-ray detector 102.

The term "source robot arm" relates to a robot for moving and holding the X-ray source 10. In an example, the robot is mounted to the floor and the X-ray source 10 is mounted at the free end of the robot. The robot may have the form of an arm with several movable joints that allow a high degree of freedom of movement.

The term "detector robot arm" relates to a robot for moving and holding the X-ray detector 102. In an example, the robot is mounted to the ceiling and the X-ray detector 102 is mounted at the free end of the robot. The robot may have the form of an arm with several movable joints that allow a high degree of freedom of movement.

The imaging system 100 as shown in Fig. 5, is configured for calibration during X-ray imaging on a regular pattern.

The term "regular pattern" relates to providing the calibration on a routine basis, like once a week, once a day, or during every run.

In an option, calibration is provided during every X-ray imaging run.

In another option, image data processing unit 106 is configured to use additional spectral information for detecting the pattern of the image data.

In a further option, X-ray source 10 is configured to provide X-ray radiation of at least a first spectrum and a second spectrum. X-ray detector 102 is configured to detect first image data relating to the first spectrum and second image data relating to the second spectrum. Further, image data processing unit 106 is configured to combine the first image data and the second image data for a detection of the calibration pattern in the image data.

The terms "first spectrum" and "second spectrum" relate to X-ray radiation provided with different spectral ranges.

In another option, X-ray source 10 is configured to provide multispectral imaging and the X-ray detector 102 is a multilayer detector configured to detect different X-ray energy levels. Further, X-ray source 10 is configured to provide radiation based on switching voltages and the X-ray detector 102 is configured to detect different X-ray energy levels in a temporally separated manner.

The term "multilayer detector" relates to a detector that comprises a plurality, i.e. at least two detector panels arranged in a stacked manner.

The term "switching voltages" relates to generating X-ray radiation with different properties by changing the voltage settings.

In an option, the image data processing unit is configured to carry out a material decomposition algorithm for distinguishing the geometrical pattern from a human anatomy as the object of interest.

In another example, not shown in detail, a calibration kit for X-ray imaging systems is provided. The kit comprises a calibration pattern and an image data processing uni. The calibration pattern is a 2D geometrical pattern made from an X-ray absorbing material. The 2D geometrical pattern comprises a plurality of graphical elements arranged in predetermined configuration allowing an image-based deduction of at least one of the group of: distance, inclination and distortion between a detector plane and the X-ray source. The calibration pattern is configured to be fixedly arranged at a source housing within an X-ray path of an X-ray generation unit that is arranged within the source housing and that is configured to generate an X-ray beam of multi-spectral radiation for spectral imaging. The housing comprises an X-ray aperture for transmitting the X-ray beam along an X-ray path towards an X-ray detector. The image data processing unit is configured to identify image portions detected by the detector, which image portions represent the calibration pattern, and to segment the calibration pattern within the image data based on the spectral imaging data. The image data processing unit is configured to provide the segmented calibration pattern for further geometrical calibration computation steps.

According to an option of the kit, the 2D geometrical pattern comprises a plurality of linear elements arranged in predetermined configuration.

In an example, the kit is provided to be able to transform a spectral X-ray imaging system into an example of the X-ray imaging system 100 shown in Fig. 5.

The calibration kit can be used for upgrading existing X-ray imaging systems which provide spectral imaging. In particular, the calibration kit can be used for upgrading existing X-ray imaging systems with separately mounted X-ray source and X-ray detector, which X-ray source and X-ray detector provide spectral imaging.

The term "kit" relates to a number of separate components that, when applied together, provide an additional function for the previously existing system.

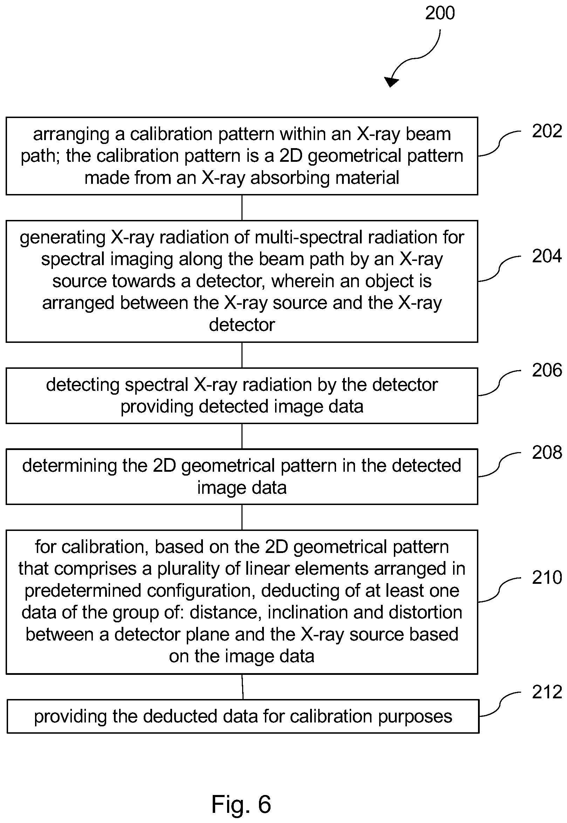

Fig. 6 shows basic steps of an example of a method 200 for calibration during X-ray imaging. The method 200 comprises the following steps:
- In a first step 202, a calibration pattern is arranged within an X-ray beam path; the calibration pattern is a 2D geometrical pattern made from an X-ray absorbing material.
- In a second step 204, X-ray radiation of multi-spectral radiation for spectral imaging is generated along the beam path by an X-ray source towards a detector; an object is arranged between the X-ray source and the X-ray detector.
- In a third step 206, spectral X-ray radiation is detected by the detector providing detected image data.
- In a fourth step 208, the 2D geometrical pattern is determined in the detected image data.
- In a fifth step 210, for calibration, based on the 2D geometrical pattern that comprises a plurality of graphical elements arranged in predetermined configuration, at least one data is deducted of the group of: distance, inclination and distortion between a detector plane and the X-ray source based on the image data.
- In a sixth step 212, the deducted data is provided for calibration purposes.

In an option of the fifth step, the 2D geometrical pattern comprises a plurality of linear elements arranged in predetermined configuration.

In another exemplary embodiment, a computer program or a computer program element is provided that is characterized by being adapted to execute the method steps of the method according to one of the preceding embodiments, on an appropriate system.

In another exemplary embodiment, a computer readable medium having stored the computer program is provided.

The computer program element might therefore be stored on a computer unit or be distributed over more than one computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on its which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to their advantage. Any reference signs in the claims should not be construed as limiting the scope.

## Claims

1. An X-ray source (10) for X-ray imaging with in-line calibration, the X-ray source comprising:
- a source housing (12);
- an X-ray generation unit (14); and
- a calibration pattern (16);
wherein the X-ray generation unit is arranged within the source housing and is configured to generate an X-ray beam (18) of multi-spectral radiation for spectral imaging along an X-ray beam path (20);
wherein the housing comprises an X-ray aperture (22) for transmitting the X-ray beam along the X-ray beam path towards an X-ray detector;
wherein the calibration pattern is a geometrical pattern (24) made from an X-ray absorbing material that is arranged at the source housing within the X-ray beam path, the geometrical pattern comprising a plurality of elements arranged in a predetermined configuration.

2. X-ray source according to claim 1, wherein the material is spectrally distinctive from human anatomy so as to enable an image-based deduction of at least one of the group of: distance, inclination and distortion between a detector plane and the X-ray source.

3. X-ray source according to claim 1 or 2, wherein the calibration pattern is fixedly arranged at the source housing within the X-ray path.

4. X-ray source according to any preceding claim, wherein the geometrical pattern is a 2D geometric pattern comprising a plurality of linear elements (26) arranged in predetermined configuration.

5. X-ray source according to claim 4, wherein the 2D geometrical pattern is a square-based line pattern with two crossing axes of symmetry; and
wherein a plurality of linear segments forms a plurality of open field segments (32).

6. X-ray source according to any preceding claim, wherein the geometrical pattern is made from Tungsten elements

7. An X-ray imaging system (100), comprising:
- an X-ray source (10) according to one of the preceding claims;
- an X-ray detector (102);
- a holding arrangement (104) for the X-ray source and the X-ray detector; and
- an image data processing unit (106);
wherein the X-ray source and the X-ray detector are mounted to the holding arrangement;
wherein the X-ray source is configured to provide spectral imaging X-ray radiation for imaging an object of interest arranged between the X-ray source and the X-ray detector;
wherein the X-ray detector is configured to receive X-ray radiation after at least partly passing through the object of interest and to detect spectral imaging data, the spectral imaging data comprising image portions representing the calibration pattern;
wherein the image data processing unit is configured to identify the image portions representing the calibration pattern and to segment the calibration pattern within the image data based on the spectral imaging data; and
wherein the image data processing unit is configured to provide the segmented calibration pattern for further geometrical calibration computation steps.

8. X-ray imaging system according to claim 7, wherein the holding arrangement comprises a robot arrangement with a source robot arm (120) and a detector robot arm (122); and
wherein the X-ray source is mounted to the source robot arm and the X-ray detector is mounted to the detector robot arm.

9. X-ray imaging system according to claim 7 or 8, wherein the imaging system is configured for calibration during X-ray imaging on a regular pattern; and
wherein calibration is provided during every X-ray imaging run.

10. X-ray imaging system according to claim 7, 8 or 9, wherein the image data processing unit is configured to use additional spectral information for detecting the pattern of the image data.

11. X-ray imaging system according to one of claims 7 to 10, wherein the X-ray source is configured to provide X-ray radiation of at least a first spectrum and a second spectrum;
wherein the X-ray detector is configured to detect first image data relating to the first spectrum and second image data relating to the second spectrum; and
wherein the image data processing unit is configured to combine the first image data and the second image data for a detection of the calibration pattern in the image data.

12. X-ray imaging system according to one of claims 7 to 11, wherein the X-ray source is configured to provide multispectral imaging and wherein the X-ray detector is a multilayer detector configured to detect different X-ray energy levels; or
wherein the X-ray source is configured to provide radiation based on switching voltages and wherein the X-ray detector is configured to detect different X-ray energy levels in a temporally separated manner.

13. X-ray imaging system according to one of claims 7 to 12, wherein the image data processing unit is configured to carry out a material decomposition algorithm for distinguishing the geometrical pattern from a human anatomy as the object of interest.

14. A method (200) for calibration during X-ray imaging, comprising the following steps:
- arranging (202) a calibration pattern within an X-ray beam path; wherein the calibration pattern is a geometrical pattern made from an X-ray absorbing material;
- generating (204) X-ray radiation of multi-spectral radiation for spectral imaging along the beam path by an X-ray source towards a detector, wherein an object is arranged between the X-ray source and the X-ray detector;
- detecting (206) spectral X-ray radiation by the detector providing detected image data;
- determining (208) the geometrical pattern in the detected image data;
- based on the determined geometrical pattern, deducting (210) of at least one data of the group of: distance, inclination and distortion between a detector plane and the X-ray source; and
- providing (212) the deducted data for calibration purposes.

15. Computer program comprising instructions which, when the program is executed by a computer, cause the computer to carry out the method of claim 14.
